(19) Europäisches Patentamt
European Patent Office
Office européen des brevets

(11) Publication number: **0 529 395 A2**

(12) **EUROPEAN PATENT APPLICATION**

(21) Application number: **92113630.5**

(22) Date of filing: **10.08.92**

(51) Int. Cl.5: **C07C 237/44**, C07C 323/19, A61K 31/165

(30) Priority: **26.08.91 US 749742**

(43) Date of publication of application: **03.03.93 Bulletin 93/09**

(84) Designated Contracting States: **AT BE CH DE DK ES FR GB GR IE IT LI LU MC NL PT SE**

(71) Applicant: **Bristol-Myers Squibb Company**
**345 Park Avenue**
**New York, N.Y. 10154(US)**

(72) Inventor: **Monkovic, Ivo**
**51 Mauro Drive**
**Durham, CT 06422(US)**
Inventor: **Wang, Lotte**
**5 King's Highway**
**North Haven, CT 06473(US)**
Inventor: **Willner, David**
**9 Raelin Road**
**Hamden, CT 06514(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86 (DE)**

(54) **Benzamide multidrug resistance reversing agents.**

(57) Benzamides of formula I

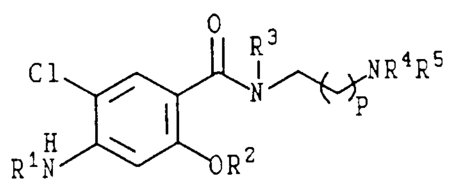

I

wherein

p is 1 to 3;

$R^1$ is hydrogen or an acyl group $R^6CO$-, in which $R^6$ is $C_{1-6}$ alkyl, $C_{3-7}$ cycloalkyl, $C_{2-6}$ alkenyl, aryl or radical of the formula

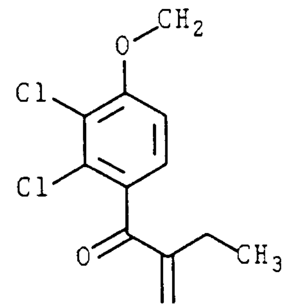

;



Rank Xerox (UK) Business Services
(3.10/3.5x/3.0.1)

$R^3$ is hydrogen or W, where W is a radical of the formula

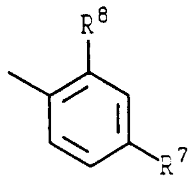

in which $R^7$ and $R^8$ each are independently $CF_3$, H or $-NO_2$, provided at least either $R^7$ or $R^8$ is $-NO_2$;

$R^2$ is W or a radical of the formula

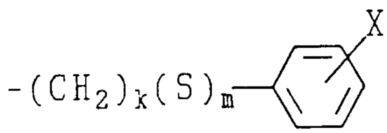

in which X is hydrogen or a halogen, k is 2 or 3, and m is 0 or 1; and

$R^4$ and $R^5$ each are independently $C_{1-6}$ alkyl are useful for the reversal of multidrug resistance to cancer drugs.

## BACKGROUND OF INVENTION

Field of Invention

This invention relates to substituted dibenz[b,f][1,4]oxazepin-11(10H)-ones useful for the reversal of multidrug resistance of cancer cells to multiple cytotoxic drugs. Thus the compounds of the instant invention can be used for adjuvant chemotherapy for neoplasias resistant to multiple drugs.

Background of Related Art

The treatment of human tumors with cytotoxic drugs is an important part of the modern clinical cancer therapy. A major obstacle to effective cancer chemotherapy is the resistance of tumor cells to antineoplastic agents. Drug resistance in human malignancies may arise from multiple mechanisms. Of particular importance is the cross resistance of cancer cells to a diverse group of lipophilic drugs with unrelated structures and functions, a phenomenon known as multidrug resistance (MDR).

A common feature detected in all MDR cells in early studies was the reduction in intracellular steady state drug accumulation relative to sensitive cells. Later, it was discovered that this phenotype was frequently associated with the increased expression of a plasma membrane glycoprotein (P-gp) of 170kDa. The implication of this protein in MDR was confirmed by its ability to confer drug resistance through transfection of cloned P-gp gene (MDR-1) into sensitive cells. See: Grace Bradley, Peter F. Juranka & Victor Ling - Mechanisms of multidrug resistance, Bioch. Biophys. Acta, **948**, pp 87-128 (1988); Jane A. Endicott & Victor Ling - The biochemistry of P-glycoprotein-mediated multidrug resistance, Ann. Rev. Biochem, **58**, pp 137-171 (1989); James M. Ford & William N. Hait - Pharmacology of drugs that alter multidrug resistance in cancer, Pharmacological Reviews, **42**, pp 155-199 (1990).

P-gp consists of two symmetrical halves, each has an ATP binding domain. Evidence suggests that it functions as an energy dependent pump with a broad range of substrate specificity. Relatively high levels of P-gp have also been found in certain normal human tissues, such as adrenal glands, kidney, colon and placenta. However, its physiological role and its natural substrate are as yet unclear. P-gp may serve to export naturally occurring toxins or xenobiotics as a detoxification mechanism. Surveys of clinical samples have found increased levels of P-gp in tumors derived from tissues which normally overexpress MDR-1 message. In addition, apparently, there is a direct correlation between the expression of P-gp with some drug refractory hematological malignancies and childhood soft tissue sarcomas, which do not normally express P-gp. See: Mace Rothenberg & Victor Ling - Multidrug Resistance: Molecular Biology and Clinical Relevance, J. Nat. Cancer Inst., **81**, pp 907-910, (1989); Helen S. L. Chan, Paul S. Thorner, George Haddad and Victor Ling - Immunohistochemical Detection of P-glycoprotein: Prognostic Correlation in Soft Tissue Sarcoma of Childhood, J. Clin. Oncol., **8**, pp 689-704 (1990). These findings support potential clinical role played by P-gp in both intrinsic and acquired MDR which ultimately render some cancer treatments inefficacious.

Several strategies have been devised to circumvent clinical MDR. One promising approach is the utilization of chemosensitizing agents which can inhibit active efflux of drugs in resistant cells. Numerous compounds including calcium antagonists, calmodulin inhibitors, and some drug analogues have shown variable abilities to reverse MDR. Most of these agents are lipophilic and may act as a substrate for the P-gp, thereby competitively inhibiting its drug efflux effect. Excellent reviews have recently been published on agents that alter multidrug resistance in cancer. See: James M. Ford & William N. Hait - Pharmacology of Drugs that Alter Multidrug Resistance in Cancer, Pharmacological Reviews, **42**, pp 155-199 (1990); David J. Stewart & William K. Evans - Non-chemotherapeutic Agents that Potentiate Chemotherapy Efficacy, Cancer Treatment Reviews, **16**, pp 1-40 (1989).

The major limiting factor to use certain MDR reversing agents in cancer patients so far is their toxicity which prevents them from reaching effective concentrations during treatment. Thus, challenge remains in the search of ideal MDR reversing agents which are more potent but less toxic and pharmacologically acceptable for clinical applications.

We have recently discovered a group of substituted benzamides with potent MDR reversing abilities. Benzamides with structures somewhat related to those of the instant invention have been disclosed in our patent to Monkovic et al., U.S. Patent No. 4,808,624 issued on Feb. 28, 1989. In the patent, the benzamides are reported to have anti-emetic activity and they are, inter alia, of the formula

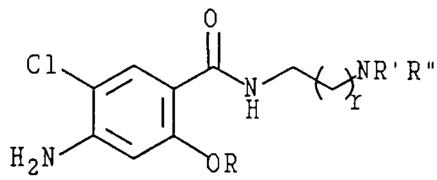

wherein

R' and R'' are the same or different and are (lower)alkyl, r is an interger from 1 to 3; R is

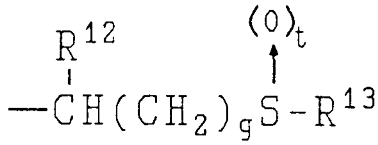

or

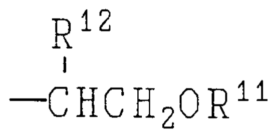

in which g is 0 to 4, t is 0 to 2, $R^{13}$ is (lower)alkyl, $R^{11}$ and $R^{12}$ are the same or different, and are hydrogen, (lower)alkyl, (lower)alkenyl, (lower)alkynl, (lower)alkoxy(lower)alkyl or cycloalkyl containing from 5 to 7 carbon atoms, inclusive, or a radical of the formula

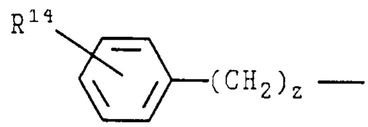

in which z is 0 to 4, and $R^{14}$ is hydrogen, halogen, hydroxy, (lower)alkyl or (lower)alkoxy, provided that, when $R^{11}$ is (lower)alkenyl or (lower)alkynyl, the unsaturated carbon atom may not be directly attached to an oxygen atom.

Another similar class of benzamides with the anti-emetic properties can also be found in U.S. Patent No. 4,820,715, issued to Monkovic et al. on April 11, 1989.

## SUMMARY OF THE INVENTION

The instant invention relates to benzamides of formula I or a pharmaceutically acceptable salt thereof

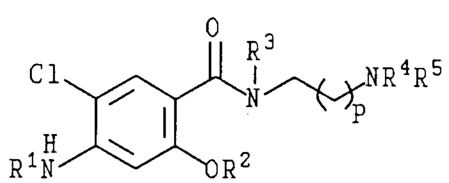

I

wherein

- p is 1 to 3;
- $R^1$ is hydrogen or an acyl group $R^6CO$-, in which $R^6$ is $C_{1-6}$ alkyl, $C_{3-7}$ cycloalkyl, $C_{2-6}$ alkenyl, aryl or radical of the formula

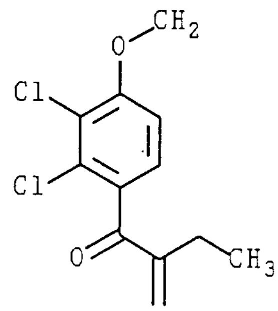

;

R3 is hydrogen or W, where W is a radical of the formula

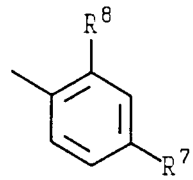

in which $R^7$ and $R^8$ each are independently $CF_3$, H or $-NO_2$, provided at least either $R^7$ or $R^8$ is $-NO_2$;

$R^2$ is W or a radical of the formula

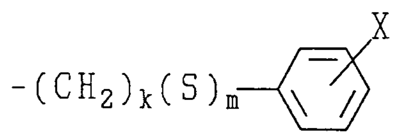

in which X is hydrogen or a halogen, k is 2 or 3, and m is 0 or 1; and

$R^4$ and $R^5$ each are independently $C_{1-6}$ alkyl.

Compounds of formula I are useful for the reversal of multidrug resistance to cancer drugs. Thus in another aspect, this invention relates to the use of compound of formula I as agents for adjuvant chemotherapy for neoplasias resistant to multiple drugs.

## DETAILED DESCRIPTION OF THE INVENTION

The instant invention relates to benzamides of formula I or a pharmaceutically acceptable salt thereof

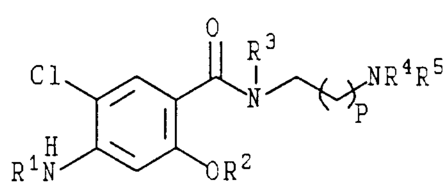

I

wherein

p is 1 to 3;

$R^1$ is hydrogen or an acyl group $R^6CO-$, in which $R^6$ is $C_{1-6}$ alkyl, $C_{3-7}$ cycloalkyl, $C_{2-6}$ alkenyl, aryl or radical of the formula

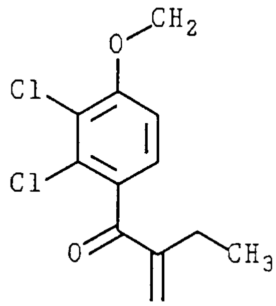 ;

$R^3$ is hydrogen or W, where W is a radical of the formula

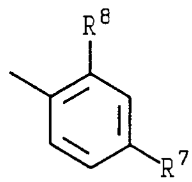

in which $R^7$ and $R^8$ each are independently $CF_3$, H or $-NO_2$, provided at least either R or $R^8$ is $-NO_2$;

$R^2$ is W or a radical of the formula

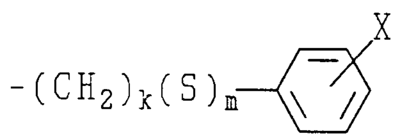

in which X is hydrogen or a halogen, k is 2 or 3, and m is 0 or 1; and

$R^4$ and $R^5$ each are independently $C_{1-6}$ alkyl.

Preferred compounds of formula I are those in which $R^1$ is hydrogen or an acyl radical $R^6CO$- selected from

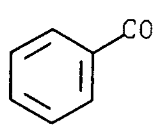 , 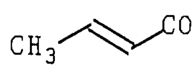 , $CH_3CO$ ,

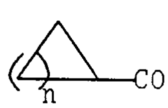 and 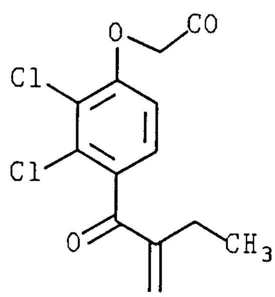

in which n is 1 to 4; p equals 1; and $R^2$ and $R^3$ are simultaneously W, or $R^3$ is hydrogen and $R^2$ is a radical of the formula

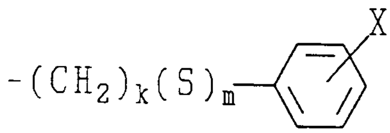

in which X, k and m are as previously defined.

Compounds of formula $I^1$ (Scheme A), which form a subset of compounds of formula I wherein $R^2$ and $R^3$ are both W and $R^1$ is hydrogen, can be made via many procedures. A preferred process is shown in Scheme A.

In Step 1 of Scheme A, the phenolic hydrogen in a compound of formula II is exchanged with a cation M to form a compound of formula III. Examples of the cation include sodium, potassium, tetrabutylammonium, and benzyltriethylammonium, to name a few. This exchange may be effected with a base such as potassium carbonate, potassium hydroxide, potassium hydride, sodium hydride, sodium hydroxide, sodium carbonate, or a quaternary ammonium hydroxide such as tetrabutylammonium hydroxide or benzyltriethylammonium hydroxide. The reaction is usally conducted in an inert organic solvent such as acetone, acetonitrile, methylene chloride, dimethylformamide (DMF), dimethylacetamide, 2-methoxyethanol, methanol, ethanol, isopropanol, or diglyme.

Step 2 of Scheme A is effected by reacting the resultant phenolic salt of formula III with preferably one to two equivalents of a compound of formula IV, in which Y is a halogen, preferably fluoro or chloro, and $R^8$ and $R^7$ have the earlier defined meanining. Step 2 is conducted in the presence of a base such as potassium carbonate and in an inert organic solvent such as acetone, acetonitrile, methylene chloride, DMF, dimethylacetamide, 2-methoxyethanol, methanol, ethanol, isopropanol, or diglyme. The preferred solvent is n-propanol or DMF. The reaction may take place with or without the application of heat; however, the reaction is preferably conducted at an elevated temperature, and even more preferably at the the reflux temperature of the solvent used. In addition to a compound of formula $I^1$, a compound of formula V may be formed in Step 2.

Scheme A

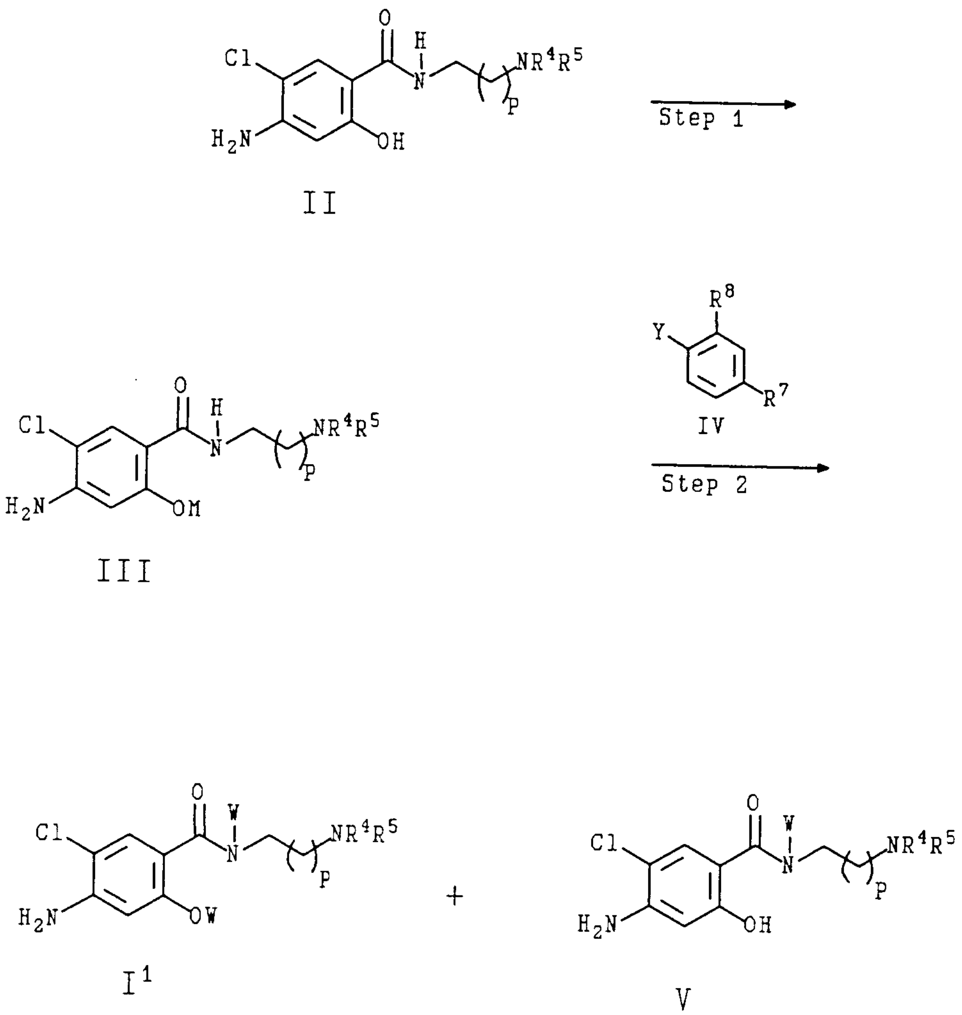


II

III

IV

$I^1$ + V

If desired, a compound of formula $I^1$ can be acylated at the free 4-amino group to replace a hydrogen atom with a radical $R^6CO$- which has the meaning as defined earlier. The acylation technique for a free aromatic amine is well established in the art. For example, a compound of formula $I^1$ can be coupled with an acid $R^6COOH$ in the presence of a dehydrating agent such as dicyclohexylcarbodiimide or 1-ethoxycarbonyl-2-ethoxy-1,2-dihydroquinoline (EEDQ). Other dehydrating agents such as the ones which appear in Synthesis, pp 453-463 (1972) may also be suitable. Alternatively, the carboxy group of $R^6COOH$ can also be converted to a reactive derivative which can be used in the N-acylation. Reactive derivatives of the carboxy group that can be used are acid halides; acid azides; mixed acid anyhydrides; acid imidazolides; active esters such as those formed with ethyl chloroformate or isobutyl chloroformate; phenylcarbamates; N-hydroxyimides such as formed with N-hydroxysuccinimide or N-hydroxyphthalimide; and those formed with hydroxybenzotriazole (HBT) or 4-methyltetrazole-5-thione; or the like active carboxy derivatives.

The synthesis of a class of compounds of formula II is well described in several patent literatures and publications. More convenient processes are those which have been used to make the starting materials for the compounds patented in U.S. Patent No. 4,808,624. Other processes which may be adopted to make compounds of formula II are summarized in the Complete Disclosure section of the same U.S. Patent.

Compounds of Formula $I^2$, which form another subset of compounds of formula I in which $R^1$ and $R^3$ are hydrogen and $R^2$ is a radical of the formula

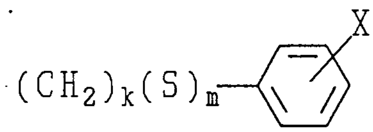

wherein X, m and k have the earlier defined meaning, can be prepared by the method of Process a in Scheme B.

Scheme B

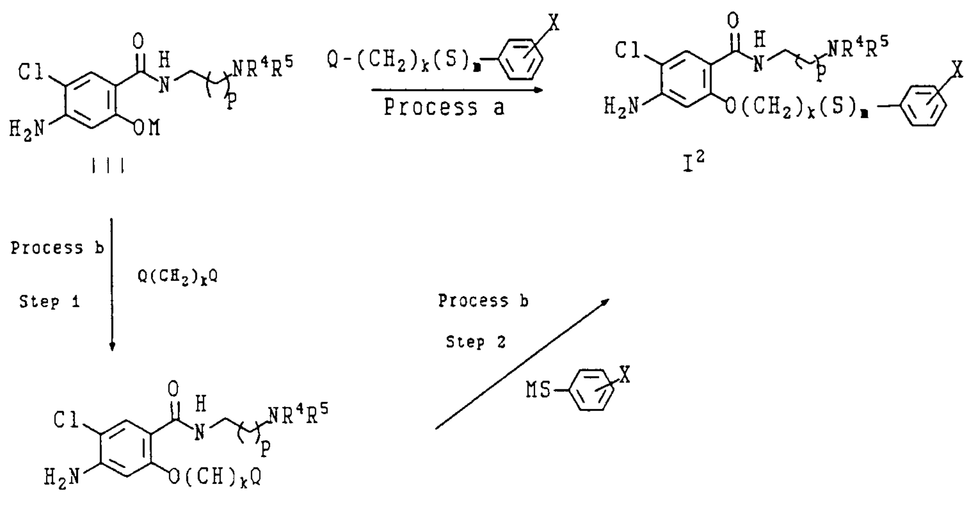


III

$I^2$

VI

In the process, a compound of formula III is treated with a compound of the formula

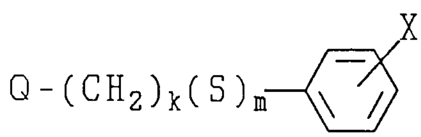

in which Q is a conventional leaving group which is well known to those skilled in the art, and examples include chloro, bromo, iodo, methanesulfonyl, toluenesulfonyl and the like. The reaction conditions, such as the temperature and the solvent, are similar to those of Step 2 in Scheme A.

Alternatively, the synthesis of a compound of formula $I^2$, when m specifically equals 1, may also be prepared by the method of Process b in Scheme B. Under the similar reaction conditions to those of Process a, the starting compound of formula III is reacted with a compound of the formula

$Q(CH_2)_kQ$

in which k and Q have the same meaning as defined previously, to afford a compound of formula VI.

In Step 2, the resulting product of formula VI is further reacted with a salt of aryl thiol of the formula

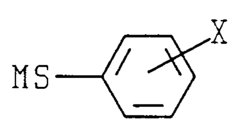

in which M is a cation with the previous definition to afford a compound of formula $I^2$ in which m now specifically equals 1. The reaction parameters of Steps 1 and 2 of Process B, Scheme B, are similar to those described for Step 2 of Scheme A.

Once again, by employing the analogous acylation technique for the free amino group in a compound of formula $I^1$, the free amino group in a compound of formula $I^2$ can be acylated with $R^6COOH$ or its reactive derivative to afford additional compounds within the scope of this invention.

Compounds of formulas $I^3$ and $I^4$, which form further subsets of compounds of formula I in which $R^3$ is W, can be made by several procedures. In a preferred embodiment, a compound of formula V, which may be concomitantly isolated from Step 2 of Scheme A, may be carried through a process analogous to Process a or b of Scheme B or Step 2 of Scheme A. Adaptation, with or without minor variations, of the earlier processes to the instant process for converting a compound of formula V to a compound of formula $I^3$ (Scheme C) shall be obvious to anyone skilled in the art.

## Scheme C

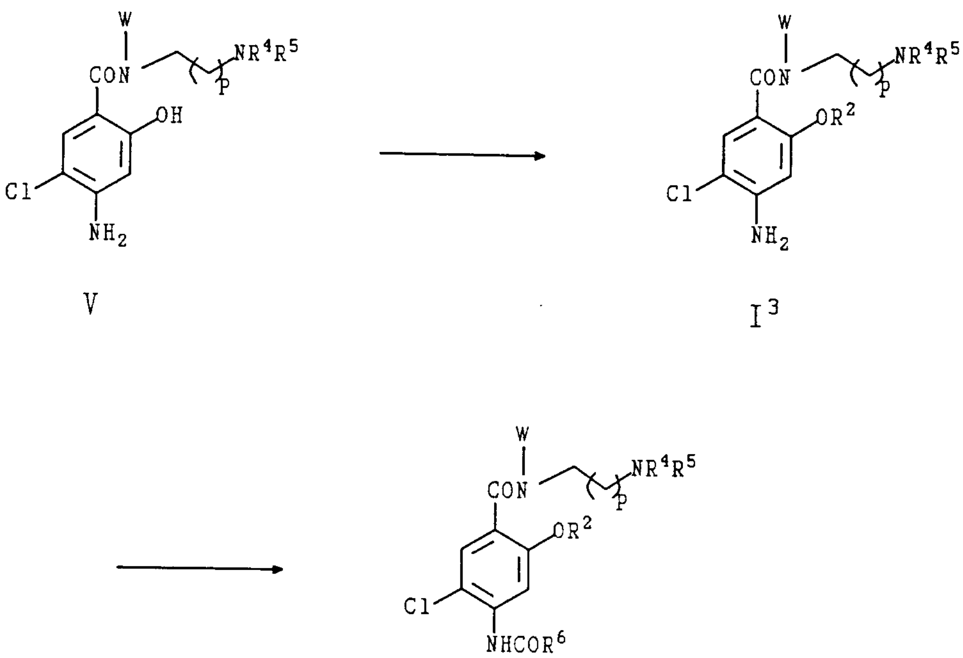

V

$I^3$

$I^4$

Again, if desired a hydrogen atom on the free 4-amino group of a compound of formula $I^3$ can be replaced with a $R^6CO$- radical by the earlier described acylation techniques to afford a compound of formula $I^4$.

In a further embodiment, a series of steps as shown in Scheme D may be employed to obtain a compound of formula $I^4$.

Scheme D

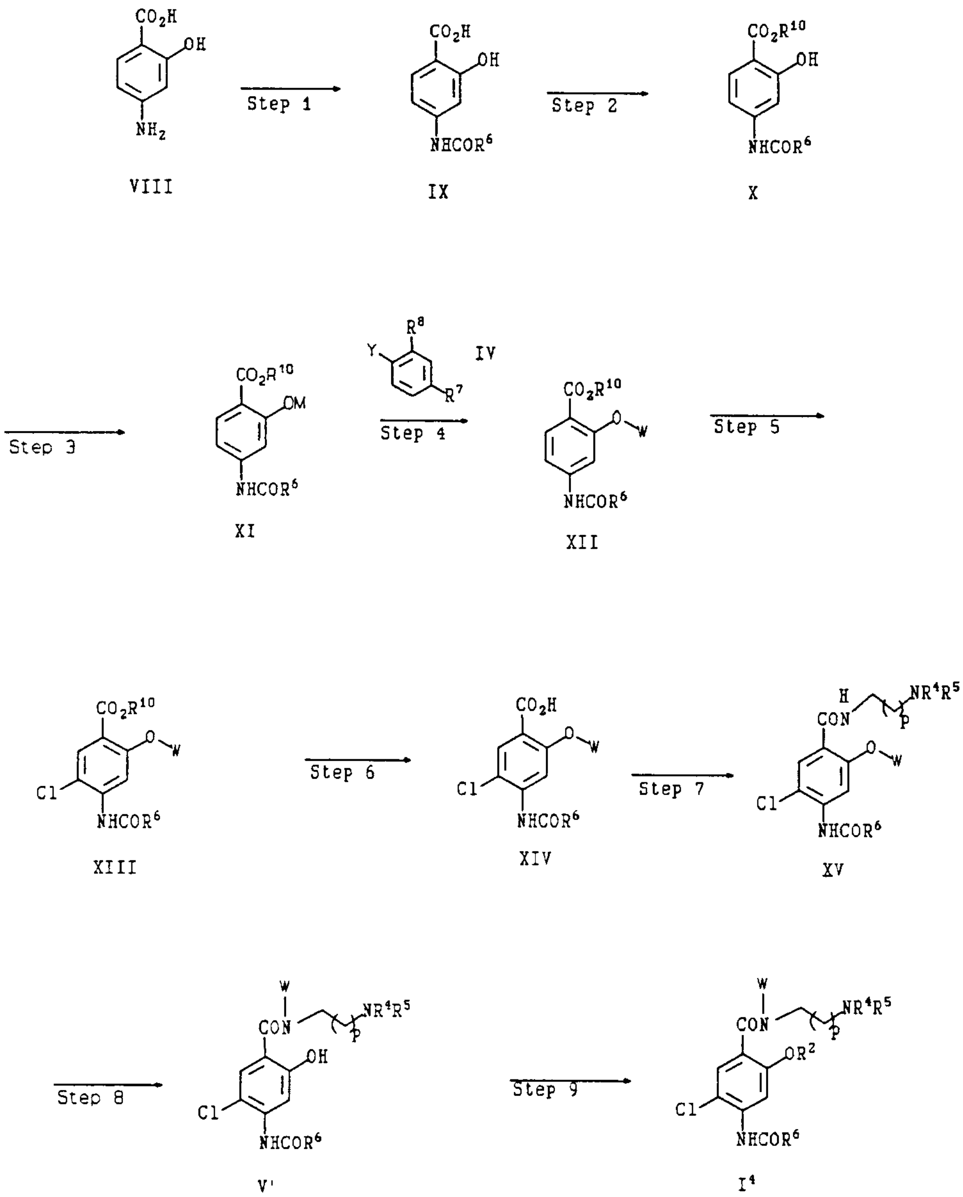


VIII IX X XI IV XII XIII XIV XV V' $I^4$

In Step 1 of Scheme D, the free amino group in compound of formula VIII is acylated with a $R^6$CO-radical. The acylation process may be similar to the methods described for the acylation of compounds of formula $I^1$, $I^2$ or $I^3$. The radical -$COR^6$ has the same meaning as defined earlier but preferably it is simply an acetyl group which can function as an amino protecting group and be easily removed by base hydrolysis in a later step. (The appropriate stage at which the acetyl protecting group may be removed can be easily ascertained by those skilled in the art.)

The carboxy group in a compound of formula IX is protected with a conventional carboxy protecting group $R^{10}$ in Step 2. Conventional carboxy protecting groups which can be employed in the present invention to block or protect the carboxylic acid function are well-known to those skilled in the art and, preferably, said groups can be removed, if desired, by methods which do not result in any appreciable destruction of the remaining portion of the molecule, for example, by chemical or enzymatic hydrolysis, treatment with chemical reducing agents under mild conditions, irradiation with ultraviolet light or catalytic hydrogenation. Examples of such readily removable carboxy-protecting groups include moieties such as

$C_{1-6}$ alkyl, diphenylmethyl (benzyhydryl), 2-naphthylmethyl, 4-pyridylmethyl, phenacyl, acetonyl, 2,2,2-trichloroethyl, silyl such as trimethylsilyl and t-butyldimethylsilyl, phenyl, ring substituted phenyl, e.g., 4-chlorophenyl, tolyl, and t-butylphenyl, phenyl $C_{1-6}$ alkyl, ring substituted phenyl $C_{1-6}$ alkyl, e.g., benzyl, 4-methoxybenzyl, 4-nitrobenzyl (p-nitrobenzyl), 2-nitrobenzyl (o-nitrobenzyl), and triphenylmethyl (trityl), methoxymethyl, 2,2,2-trichloroethoxycarbonyl, benzyloxymethyl, $C_{1-6}$alkanoyloxy $C_{1-6}$ alkyl such as acetoxymethyl, propionyloxymethyl, $C_{2-6}$ alkenyl such as vinyl and allyl. Other suitable carboxy protecting groups well known in the art can be found in "Protective Groups in Organic Synthesis", Theodora W. Greene (John Wiley & Sons, 1981), Chapter 5. A particularly advantageous carboxy protecting group is allyl.

In step 3, the free phenolic hydrogen is exchanged with a cation M by a procedure analogous to that described for Step 1 of Scheme A. A compound of formula XI thus formed is reacted with a compound of formula IV in Step 4. The conditions employed in Step 4 are analogous to those employed for Step 2 in Scheme A.

A compound of formula XII thus formed is chlorinated in Step 5. The chlorination can be achieved by a standard manner of chlorinating an aromatic ring such as with sulfuryl chloride in methylene chloride, chlorine in acetic acid, N-chlorosuccinimide or other suitable methods of chlorination.

Step 6 involves the removal of a conventional carboxy protecting group. When the protecting group is allyl, it may be removed with tris(dibenzylideneacetone)dipalladium (0) and triphenylphosphine.

Step 7 involves condensation of an amine $H_2NCH_2(CH_2)_pNR^4R^5$ with a benzoic acid derivative of formula XIV to form a benzamide of formula XV. Several methods are available for such a condensation. For example, the Complete Disclosure section of U.S. Patent No. 4,808,624 discloses a number of representative methods.

Treatment of a compound of formula XV with base promotes the rearrangement of W in Step 8. Heat may be applied to facilitate the process.

Once again, the method of transformation of a compound of formula V' to a compound of formula $I^4$ in Step 9 is similar to Process a or b of Scheme B or Step 2 of Scheme A.

In a compound of formula $I^4$, when $R^aCO$- is acetyl, it can be removed by base hydrolysis to afford a compound of formula $I^3$. If desired, a hydrogen atom of the free 4-amino group may once again be replaced by a radical $R^aCO$- which is now different from acetyl.

In another embodiment, under the similar conditions described for Step 7 of Scheme D, a compound of formula XIV can be condensed with an amine $HWNCH_2(CH)_pNR^4R^5$, in which W, p, $R^5$ and $R^6$ have the meaning as defined earlier, to afford a compound of formula $I^5$. In a compound of formula $I^5$, when $R^aCO$- is acetyl, it can be removed by base hydrolysis to afford a compound of formula $I^1$ (Scheme E). If desired, a hydrogen atom of the newly-freed 4-amino group may now be replaced with a radical $R^aCO$- which is different from acetyl.

Scheme E

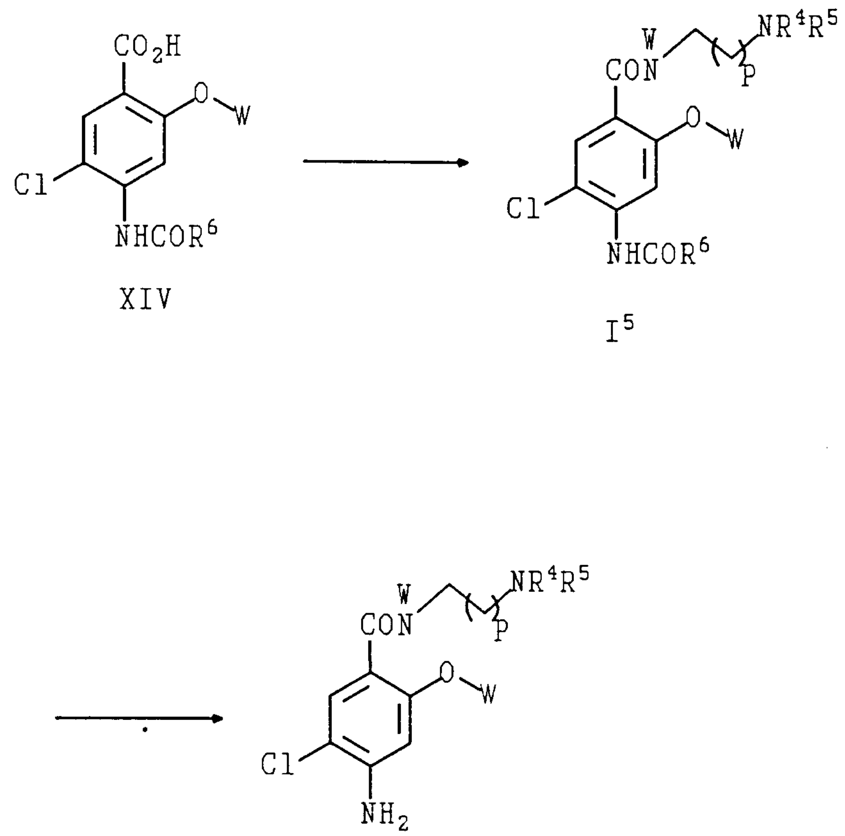

XIV

$I^5$

$I^1$

The amine of the formula $HWNCH_2(CH)_pNR^4R^5$ may be readily prepared by reacting a primary amine of the formula $H_2NCH_2(CH)_pNR^4R^5$ with a compound of formula IV. The addition may be effected in an inert organic solvent such as acetone, acetonitrile, methylene chloride, DMF, dimethylacetamide, 2-methoxyethanol, methanol, ethanol, isopropanol, or diglyme. The reaction may take place with or without the application of heat; however, the reaction is preferably conducted at an elevated temperature, and even more preferably at the reflux temperature of the solvent used.

In the instant application, the numbers in subscript after the symbol "C" define the number of carbon atoms a particular group could contain. For example, $C_{1-6}$ alkyl refers to straight and branched chain alkyl groups with one to six carbon atoms and such groups include methyl, ethyl, n-propyl, isopropyl, n-butyl, t-butyl, n-pentyl, n-hexyl, 3-methylpentyl and the like alkyl groups; $C_{2-6}$ alkenyl refers to straight or branched alkenyl groups such as vinyl, allyl, 1-propenyl, isopropenyl, 1-butenyl, 2-butenyl, 3-butenyl, methallyl, 1,1-dimethylallyl, 1-hexenyl, 2-hexenyl and the like groups; cyclic $C_{3-7}$ alkyl refers to groups such as cyclopropyl, cyclobutyl, cyclopentyl, cyclohexyl, cycloheptyl, cyclopropylmethyl, cyclopropylethyl, cyclopropylpropyl, cylcobutylmethyl, cyclobutylethyl, cyclopentylmethyl and the like groups; aryl group refers to unsubstituted phenyl or phenyl independently substituted with one to three halogen, $C_{1-6}$ alkyl, $C_{1-6}$ alkyloxy or $C_{1-6}$ alkylthio such as 4-methylphenyl, 2,3-dimethoxyphenyl, 2-methyl-3-ethoxylphenyl, 4-t-butoxyphenyl, 4-methylthio-3-fluorophenyl, 2,4-dichlorophenyl, 2-chloro-4-bromophenyl and the like groups; $C_{1-6}$ alkyloxy (alkoxy) refers straight or branched alkyloxy groups such as methoxy, ethoxy, n-propoxy, i-propoxy, n-butoxy, t-butoxy, n-pentyloxy, n-hexyloxy, 3-methylpentyloxy, to name a few; $C_{1-6}$ alkylthio refers straight or branched alkylthio groups such as methylthio, ethylthio, n-propylthio, i-propylthio, n-butylthio, t-butylthio, n-pentylthio, n-hexylthio, 3-methylpentylthio and the like groups; and halogen refers to fluorine, chlorine, bromine, or iodine.

The structural formulae as drawn herein are believed to best represent the structures of compounds of the present invention. However, some compounds within the scope of the invention may exist as other tautomeric forms, in which hydrogen atoms are transposed to other parts of the molecules and the chemical bonds between the atoms of the molecules are consequently rearranged. It should be understood that the structural formulae represent all tautomeric forms, insofar as they may exist.

DESCRIPTION OF SPECIFIC EMBODIMENTS

The specific examples which follow illustrate the synthesis of representative compounds of the instant invention and are not construed as limiting the invention in sphere or scope. The methods may be adopted to variations in order to produce compounds embraced by this invention but not specifically disclosed. Further, variations of the methods to produce the same compounds in somewhat different fashion will also be evident to one skilled in the art.

All temperatures are understood to be in Centigrade (C) when not specified. The nuclear magnetic resonance (NMR) spectral characteristics refer to chemical shifts ($\delta$) expressed in parts per million (ppm) versus tetramethylsilane (TMS) as reference standard. The relative area reported for the various shifts in the proton NMR spectral data corresponds to the number of hydrogen atoms of a particular functional type in the molecule. The nature of the shifts as to multiplicity is reported as broad singlet (bs), broad doublet (bd), broad triplet (bt), broad quartet (bq), singlet (s), multiplet (m), doublet (d), quartet (q), triplet (t), doublet of doublet (dd), doublet of triplet (dt), and doublet of quartet (dq). The solvents employed for taking NMR spectra are DMSO-$d_6$ (perdeuterodimethysulfoxide), $D_2O$ (deuterated water), $CDCl_3$ (deuterochloroform) and other conventional deuterated solvents. The infrared (IR) spectral description include only absorption wave numbers ($cm^{-1}$) having functional group identification value.

Celite is a registered trademark of the Johns-Manville Products Corporation for diatomaceous earth.

The abbreviations used herein are conventional abbreviations widely employed in the art. Some of which are:

| | |
|---|---|
| MS | : Mass spectrometry |
| HRMS | : High resolution mass spectrometry |
| DMF | : Dimethylformamide |
| Ac | : Acetyl |
| ADR | : Adriamycin |
| ActD | : Actinomycin D |
| DMSO | : dimethyl sulfoxide |
| Ph | : phenyl |

Example 1

4-Amino-5-chloro-N-[2-(diethylamino)ethyl]-N-[2-nitro-4-(trifluorormethyl)phenyl]-2-[(2-nitro-4-(trifluoromethyl)phenoxy]benzamide (Ia)

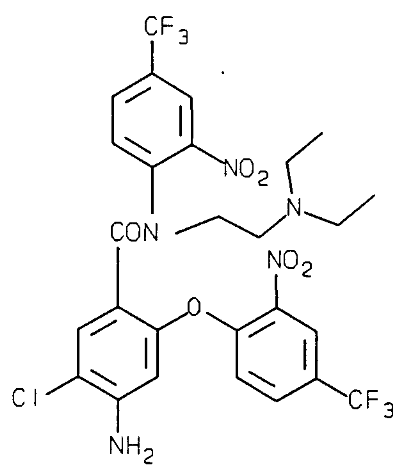

A suspension of 60% sodium hydride in mineral oil (1.76 g, 44 mmol, washed with n-pentane) under nitrogen was treated with n-propanol (80 ml). To this was added 4-amino-5-chloro-N-[2-(diethylamino)ethyl]-2-hydroxybenzamide hydrochloride (6.44 g, 20 mmol) and 4-chloro-3-nitrobenzotrifluoride (4.51 g, 20 mmol). The mixture was heated under reflux for 6 h and then concentrated in vacuo. The residue was partitioned between aqueous $NaHCO_3$ solution and a 1:1:1 mixture of dichloromethane, ether and n-hexane. The organic phase was washed with 1N NaOH, water and than treated with 20 ml of 1 N HCl. A precipitated solid was collected by filtration and washed with acetone to give 1.65 g of crude 3-amino-2-chloro-10-[2-(diethylamino)ethyl]-7-(trifluoromethyl)dibenz[b,f][1,4]oxazepin-11(10H)-one dihydrochloride, as a light yellow solid. The mother liquors were combined, neutralized with an aqueous solution of $NaHCO_3$ and extracted into $CH_2Cl_2$. The extract was dried and concentrated and the residue was chromotographed on silicia using $CH_2Cl_2$ with 2-8% MeOH as the eluent, to give the following three fractions.

a) The first fraction, 430 mg of a yellow amorphous solid, was the title compound, 4-amino-5-chloro-N-[2-(diethylamino)ethyl)-N-[2-nitro-4-(trifluoromethyl)phenoxy]benzamide (Ia), mp >60 ° C.

$^1$H NMR ($CDCl_3$) δ 8.0-8.3 (m, 3H), 7.7-7.9 (m, 4H), 7.02 (s, 1H), 6.11 (s, 1H), 4.26 (m, 2H), 2.2-2.8 (m, 6H), 0.80 (m, 6H);

MS(m/e) 663;

Anal, Calcd for $C_{27}H_{24}ClF_6N_5O_6$: C 48.84, H 3.64, N 10.55 Found: C 50.75, H 4.00, N 9.85

b) The second fraction, 359 mg of a yellow solid which had identical Rf value as the above-described yellow solid (1.65 g), had spectral data which were consistent with structure of 3-amino-2-chloro-10-[2-(diethylamino)ethyl]-7-(trifluoromethyl)dibenz[b,f][1,4]oxazepin-11(10H)-one (free base).

$^1$H NMR ($CDCl_3$) δ 7.72 (s, 1H), 7.63 (d, 1H), 7.42 (d, 1H), 4.42 (s, 1H), 6.52 (s, 1H), 4.45 (s, 2H), 4.09 (+2H), 2.77 (t, 3H), 2.52 (q, 4H), 6.96 (t, 6H);

Ms (m/e) 428 corresponds to $M+H^+$.

The sample was treated with anhydrous HCl in MeOH, and the product was combined with previously obtained solid (1.65 g) and recrystallized from MeOH-$Et_2O$ to give 1.85 g of a light beige solid, mp >130 ° C.

Analysis, Calcd for $C_{20}H_{21}ClF_3N_3O_2$•2 HCl: C 47.97, H 4.63, N 8.39 Found: C 47.74, H 4.58, N 8.33

c) The third fraction was 68 mg of 4-amino-5-chloro-N-[2-(diethylamino)ethyl]-2-hydroxy-N-[2-nitro-4-(trifluoromethyl)phenyl] benzamide (Ia) isolated as a yellow solid, mp >100 ° C.

$^1$H NMR ($CDCl_3$) δ 8.10 (s, 1H), 7.96 (s, 2H), 6.38 (s, 1H), 6.21 (s, 1H), 4.39 (s, 2H), 4.2-4.4 (m, 2H), 2.7-3.2 (m, 6H), 1.2 (m, 6H);

MS (m/e) 474.

Example 2

4-Amino-5-chloro-N-[2-(diethylamino)ethyl]-2-[(3-phenylthio)propoxy]benzamide (Ib)

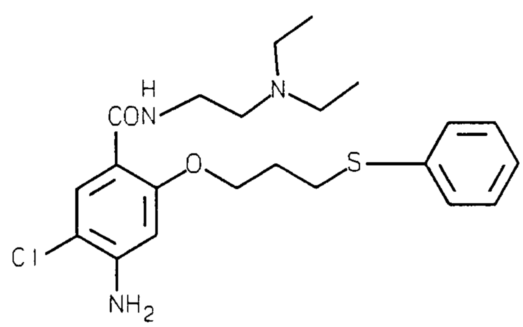

To a solution of tetra-n-buthylammonium salt of 4-amino-5-chloro-N-[2-(diethylamino)ethyl]-2-hydroxybenzamide (1.2 g, 2.276 mmol) in 20 ml $CH_3CN$ was added 1,3-dibromopropane (1.0 g, 5.0 mmol) and the mixture stirred for 30 min and heated to reflux for 1 h. After cooling, the mixture was partitioned between aqueous $NaHCO_3$ and a 1:1:1 mixture of $CH_2Cl_2$, ether and n-pentane. The organic phase was washed several times with water and then extracted with 1N HCl. The extract was neutralized with an aqueous $NaHCO_3$ solution and the product extracted into $CH_2Cl_2$. The $CH_2Cl_2$ phase was dried and concentrated in vacuo to give 560 mg (60%) of 4-amino-2-(3-bromopropoxy)-5-chloro-N-[2-(diethylamino)ethyl]benzamide (VIa) as a light yellow solid.

$^1H$ NMR ($CDCl_3$) δ 8.10 (s, 1H), 7.92 (s, 1H), 6.3 (s, 1H), 4.3 (s, 2H), 4.19 (t, 2H), 3.53 (t, 3H), 2.55 (m, 6H), 2.47 (m, 2H), 0.98 (t, 6H).

The above compound VIa was used in the next step without further purification as follows: To a solution of thiophenolate, prepared from 56 mg of 60% sodium hydride in mineral oil (1.4 mmol) and thiophenol (150 mg, 1.36 mmol) in 8 ml absolute ethanol, was added a solution of compound VIa (550 mg, 1.348 mmol) in 5 ml ethanol. The mixture was stirred for 5 min, heated to reflux for 10 min and then concentrated in vacuo. The residue was partititioned between $CH_2Cl_2$ and water. The organic phase was washed with water, dried and concentrated in vacuo to give 570 mg of the title compound (96% yield from 4-amino-5-chloro-N-[2-(diethylamino)ethyl]-2-hydroxybenzamide) as an off-white solid, mp 92-8°C. This compound was filtered over a short silica column, and the product was recrystallized from $CH_2Cl_2$/n-pentane to give 480 mg of a white solid, mp 99-103°C.

Anal Calcd for $C_{22}H_{30}ClN_3O_2S$: C, 60.60, H, 6.93, N, 9.64 Found: C, 60.95, H, 6.94, N, 9.35

$^1H$ NMR ($CDCl_3$) δ 8.09 (s, 1H), 7.98 (bs, 1H), 7.15-7.34 (m, 5H), 6.22 (s, 1H), 4.27 (s, 2H), 4.14 (t, 2H), 3.47 (2, 2H), 3.08 (t, 2H), 2.54 (m, 6H), 2.17 (m, 2H), 0.96 (t, 6H).

Example 3

4-Amino-5-chloro-N-[2-(diethylamino)ethyl]-2-[3-(phenyl)pronoxy]benzamide (Ic)

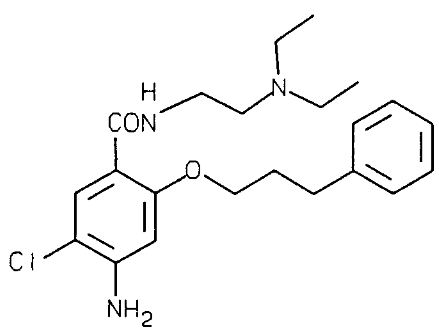

A mixture of tetra-n-buthylammonium salt of 4-amino-5-chloro-N-[2-(diethylamino)ethyl]-2-hydroxybenzamide (1.055 g, 2 mmol) and 1-bromo-3-phenylpropane (400 mg, 2 mmol) in 8 ml of acetonitrile was refluxed for 1h. After cooling, the mixture was partitioned between water and ethylacetate. The organic phase was washed with water (5 x 20 ml), dried and concentrated in vacuo. The residue was recrystallized from ether/n-pentane to give 590 mg (73%) of the title compound as a while solid mp, 105-107°C.

Anal Calcd for $C_{22}H_{30}ClN_3O_2$: C65.41, H7.48, N10.40 Found: C65.59, H7.47, N10.12;

$^1H$ NMR ($CDCl_3$) δ 8.11 (s, 1H), 8.07 (bs, 1H), 7.14-7.30 (m, 5H), 6.16 (s, 1H), 4.26 (s, 2H), 3.99 (t, 2H), 3.99 (t, 2H), 3.49 (q, 2H), 2.78 (t, 2H), 2.59 (t, 2H), 2.51 (q, 4H), 2.18 (m, 2H), 0.95 (5, 6H).

Example 4

4-Amino-5-chloro-N-[2-(diethylamino)ethyl]-2-[2-(4-chlorophenylthio)ethoxy]benzamide (Id)

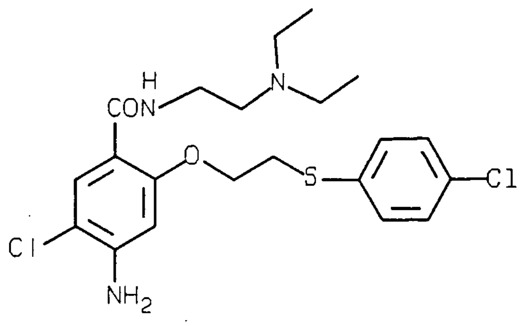

A mixture of tetrabuthylamonium salt of 2-amino-5-chloro-N-[2-(diethylamino)ethyl]-2-hydroxybenzamide (1.054 g, 2 mmol) and 2-chloroethyl-p-chlorophenylsulfide (414 mg, 2 mmol) in 2 ml of acetonitrile was heated under reflux for 24 h and than concentrated in vacuo. The residue was partitioned between ethylacetate and water. The organic phase was washed several times with water, dried and concentrated in vacuo. The residue was purified by chromatography on silica using $CH_2Cl_2$/5 to 10% MeOH as the eluent to give 388 mg (42.5%) of the title compound as a white solid, mp 122-126 ° C.

$^1$H NMR ($CDCl_3$) δ 8.17 (t, 1H), 8.06 (s, 1H), 7.29 (m, 5H), 6.14 (s, 1H), 4.36 (s, 2H), 4.12 (t, 2H), 3.57 (q, 2H), 3.29 (t, 2H), 2.78 (t, 1H), 2.70 (q, 4H), 1.07 (t, 6H).

HRMS calcd for $C_{21}H_{27}N_3Cl_2O_2S$, 456.1279.
Found: 456.1278.

Example 5

2-Acetamido-5-chloro-N-[2-(diethylamino)ethyl]-2-[2-(phenylthio)ethoxy]benzamide (Ie)

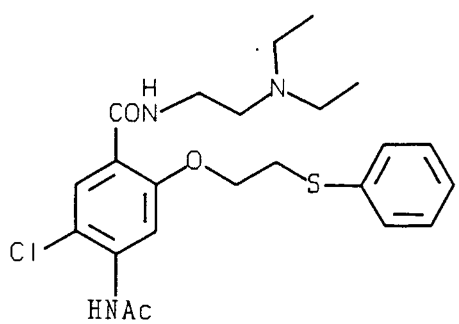

A solution of 2-amino-5-chloro-N-[2-(diethylamino)ethyl]-2-[2-(phenylthio)ethoxy]benzamide (842 mg, 2 mmol) in 5 ml acetic anhydride was heated to reflux for 2 h and than concentrated in vacuo. The residue was chromotographed on silica using $CH_2Cl_2$/5 to 10% MeOH as the eluent to give 578 mg (62.3%) of the title compound as a white solid, mp 103-106 ° C.

$^1$H NMR ($CDCl_3$) δ 7.86 (s, 1H), 7.28 (m, 5H), 4.89 (s, 3H), 4.28 (t, 2H), 3.57 (t, 2H), 3.43 (t, 2H), 2.84 (t, 2H), 2.73 (q, 4H), 1.88 (s, 2H), 1.11 (t, 6H);

HRMS Calcd for $C_{23}H_{30}N_3ClO_3S$, 464.1775. Found: 464.1785.

Example 6

5-Chloro-4-crotonylamino-N-[2-(diethylamino)ethyl]-2-[2-(phenylthio)ethoxy]benzamide (If)

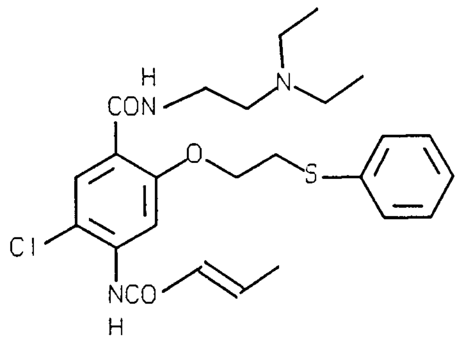

To a stirred cooled (0 ° C) solution of 4-amino-5-chloro-N-[2-(diethylamino)ethyl]-2-[2-(phenylthio)ethoxy]-benzamide (210 mg, 0.5 mmol) in pyridine (5 ml) was added crotonyl chloride (105 mg, 1.0 mmol). The mixture was stirred at a cold temperature for 2 h and then partitioned between an aqueous $NaHCO_3$ solution and ehtyl acetate. The organic phase was washed with water, dried and concentrated in vacuo. The residue was purified on preparative silica plate using $CH_2Cl_2$/15% MeOH as a mobile phase to give 115 g (47%) of the title compound as a yellow solid, mp 84-85 ° C.

$^1H$ NMR ($CDCl_3$) δ 8.34 (t, 1H), 8.27 (s, 1H), 8.175 (s, 1H), 7.69 (s, 1H), 7.341 (m, 2H), 7.210 (m, 3H), 7.134 (m, 1H), 5.940 (d of d, 1H), 4.244 (t, 2H), 3.570 (q, 2H), 3.333 (t, 2H), 2.761 (t, 2H), 2.660 (q, 4H), 1.88 (d of d, 3H), 1.039 (t, 6H);

HRMS Calcd for $C_{25}H_{32}N_3O_3SCl$, 490.1931
Found: 490.1936.

Example 7

5-Chloro-N-[2-(diethylamino)ethyl]-4-ethacrynylamino-2-[2-(phenylthio)ethoxy]benzamide (Ig)

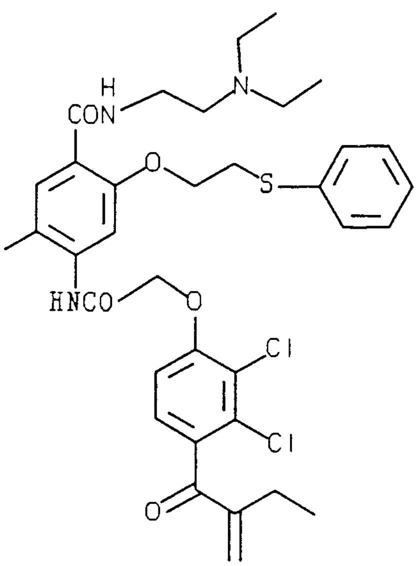

Ethacrynic acid (606 mg, 2 mmol) in 10 ml $CH_2Cl_2$ was activated by reaction with 0.5 N solution of chloromethylene-dimethylimmonium chloride in 4 ml of chloroform (Arnolds reagent, prepared by reaction of oxally chloride and DMF in chloroform) for 30 minutes. To this was added a solution of 4-amino-5-chloro-N-[2-(diethylamino)ethyl]-2-[2-(phenylthio)ethoxy]benzamide (422 mg, 1 mmol) in 5 mL dichloromethane at

0°C and the mixture was stirred for 40 minutes. To this was added dimethylamine (405 mg, 4 mmol) and stirring continued for 16 hours at room temperature. The mixture was partitioned between dichloromethane and aqueous sodium bicarbonate solution. The organic phase was concentrated in vacuo and the residue chromatographed on silica using dichloromethane/5% methanol as eluant to give 470 mg (66.5%) of the title compound as a yellow amorphous solid.

$^1H$ NMR ($CDCl_3$) δ 8.27 (t, 1H), 8.21 (s, 1H), 8.19 (s, 1H), 7.34 (s, 1H), 7.31 (s, 1H), 7.20 (m, 4H), 6.83 (d, 1H), 5.88 (s, 1H), 5.51 (s, 1H), 4.66 (s, 2H), 4.23 (t, 2H), 3.49 (q, 2H), 3.32 (t, 2H), 2.656 (t, 2H), 2.50 (q, 3H), 2.39 (q, 2H), 1.098 (t, 5H), 0.942 (t, 6H).

HRMs Calcd for $C_{34}H_{38}N_3O_5SCl_3$, 706.1676.
Found: 706.1684.

Example 8

4-Amino-5-chloro-N-[2-(diethylamino)ethyl]-2-(4-nitrophenoxy)-N-(4-nitrophenyl)benzamide (Ii)

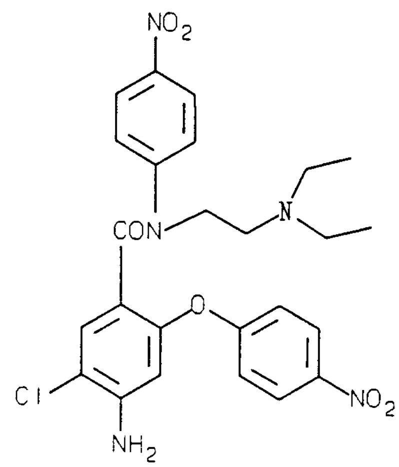

To a stirred solution of tetrabutylammonium salt of 4-amino-5-chloro-N-[2-(diethylamino)ethyl]-2-hydroxybenzamide (1.05 g, 2 mmol) in DMF (10 ml) was added 4-fluoronitrobenzene (2 mmol, 282 mg). The mixture was stirred for 2 h and then DMF was removed in vacuo. The residue was partitioned between water and ethyl acetate, and the organic phase was washed twice with water, dried and concentrated in vacuo. The residue was chromotographed on silica using $CH_2Cl_2$ with 2-10% MeOH as the eluent to give 480 mg (45.5%) of the title compound as a yellow solid, mp 85-89°C.

$^1H$ NMR δ 8.08 (d of d, 4H), 7.318 (s, 1H), 7.217 (d, 2H), 6.76 (d, 2H), 6.044 (s, 1H), 4.24 (s, 2H), 3.807 (t, 2H), 2.483 (t, 2H), 2.375 (q, 4H), 0.840 (t, 6H);

HRMS Calcd for $C_{25}H_{26}N_5O_6Cl$, 528.1650.
Found: 528.1639

Example 9

BIOLOGICAL ASSAY

Cell Culture: HCT116/VM46 cells were selected from human colon carcinoma HCT116 cell line for resistance to VM26 and MCF-7/ADR cells were selected from human breast carcinoma MCF-7 cell line for resistance to Adriamycin. Both cell types exhibit MDR phenotype and overexpress high levels of MDR-1 mRNA. The cell lines were grown in tissue culture flasks containing McCoy's 5A medium and 10% fetal bovine serum. Cells were maintained at 37°C in a humidified atmosphere containing 5% $CO_2$ and subcultured every 5 days.
Cytotoxic Assay: The cells were seeded in 96 well microtiter plates at $5x10^3$ cells per well and allowed to grow for 24 hours at 37°C. Cells were then incubated with decreasing amounts of antitumor agents: Adriamycin (100 μM, maximal concentration) or actinomycin D (17.6 ng/ml), for MCF-7 and HCT-116 cells,

respectively. Chemosensitizers were added at variable concentrations ranging from 0.08 μM to 40 μM. In parallel, verapamil was used as positive control at the same concentrations. After 48 hours of incubation, the cells were washed, fixed and stained with crystal violet. Absorbance was read by Molecular Devices' microtiter plate reader at the wavelength of 595nM. The $IC_{50}$ value (50% inhibition of cell growth) was determined from relative survival rates resulted from two to three independent experiments. The term "fold resistance" was defined as the ratio of the $IC_{50}$ for antitumor drug in the presence or absence of chemosensitizer in resistant cells divided by the $IC_{50}$ for antitumor drug in its sensitive counterpart. This value provides an estimate of the apparent potency of each reversing agent in enhancing drug effect.

Table I and II show a few representative chemosensitizers of the instant invention which showed higher MDR reversing activity compared to that of verapamil in human colon carcinoma HCT-116 resistant and in human breast carcinoma MCF-7 resistant cells, respectively.

TABLE I

| MDR Reversing Effects Of Chemosensitizers In Human Colon Carcinoma HCT-116 Cells | | | |
|---|---|---|---|
| Cell Line | Compound (0.4 μM) | ActD $IC_{50}$ (ng/ml) | Fold Resistance |
| HCT116 | - - | 0.5 | 1.0 |
| HCT-116/VM46 | - - | 10 | 20.0 |
| HCT-116/VM46 | If | 2.4 | 4.8 |
| HCT-116/VM46 | Ic | 4.9 | 9.8 |
| HCT-116/VM46 | Ii | 4.9 | 9.8 |
| HCT-116/VM46 | Ia | 5.5 | 11.0 |
| HCT-116/VM46 | Verapamil | 5.8 | 11.6 |

TABLE II

| MDR Reversing Effects of Chemosensitizers in Human Breast Carcinoma MCF-7 Cells | | | |
|---|---|---|---|
| Cell Line | Compound (0.67 μM) | ADR $IC_{50}$ (μM) | Fold Resistance |
| MCF-7 | - - | 0.36 | 1 |
| MCF-7/ADR | - - | 50 | 138.9 |
| MCF-7/ADR | If | 10.3 | 28.6 |
| MCF-7/ADR | Verapamil | 23 | 63.9 |

The foregoing tests revealed that the compounds of the instant invention are useful for the reversal of MDR to cancer drugs. Thus this invention also relates to the use of compound of formula I as agents for adjuvant chemotherapy for neoplasias resistant to multiple drugs.

Compounds of formula I may form pharmaceutically acceptable acid addition salts. Said salts are those in which anion does not contribute significantly to the toxicity of the salt and are compatible with the customary pharmaceutical vehicles and adapted for oral or parenteral adimistration. The pharmaceutically acceptable acid addition salts include the salts of compounds of formula I with mineral acids such as hydrochloric acid, hydrobromic acid, phosphoric acid and sulfuric acid; with organic carboxylic acids or organic sulfonic acids such as acetic acid, citric acid, maleic acid, succinic acid, benzoic acid, tartaric acid, fumaric acid, mandelic acid, ascorbic acid, malic acid, methanesulfonic acid, isethionic acid, p-tolenesulfonic acid and other acids known and used in the galenic pharmacy. Thus this invention further relates to a pharmaceutically acceptable salt of a compound of formula I.

The mode of systemic administration, dosage, and dosage regimen must in each case be carefully adjusted by utilization of sound professional judgment and consideration of the age, weight and condition of the recipient. Generally, the daily dose will be from about 0.1 g to about 10 g, preferably 0.5 g to 5 g, when given orally. In some instances, a sufficient therapeutic effect can be obtained at lower doses while in others, larger doses will be required. As is apparent to one skilled in clinical pharmacology, the amount of a formula I compound comprising the daily dose may be given in a single or divided dose, taking into account those principles understood by the skilled practitioner and necessary for his practice of the art.

The term "systemic administration" as used herein refers to oral, sublingual, buccal, nasal, dermal, rectal, intramuscular, intravenous, and subcutaneous routes. Generally, it will be found that should a compound of the present invention is administered orally, a slightly larger quantity of the active drug may be required to produce the same effect as a somewhat smaller quantity when given parenterally. In accordance with good clinical practice, it is preferred to administer the instant compounds at a concentration level which will produce effective beneficial effects without causing any harmful or untoward side effects.

Therapeutically, the instant compounds are generally given as pharmaceutical formulations comprised of an effective MDR reversing amount of a compound of formula I or a pharmaceutically acceptable acid addition salt thereof and a pharmaceutically acceptable carrier. Pharmaceutical formulations for effecting such treatment will contain a major or minor amount (e.g. from 95% to 0.5%) of at least one compound of the present invention in combination with pharmaceutical carrier, the carrier comprising one or more solid, semi-solid, or liquid diluent, filler and formulation adjutant which is non-toxic, inert and pharmaceutically acceptable. Such pharmaceutical formulations are preferably in dosage unit forms; i.e. physically discrete units having a pre-determined amount of the drug corresponding to a fraction or multiple of the dose which is calculated to produce the desired therapeutic response. In usual practice, the dosage units contain 1, 1/2, 1/3, or less of a single dose. A single dose preferably contains an amount sufficient to produce the desired therapeutic effect upon administration at one application of one or more dosage units according to the pre-determined dosage regimen, usually a whole, half, third, or less of the daily dosage administered once, twice, three or more times a day. It is envisioned that other therapeutic agents can also be present in such a formulation. Pharmaceutical formulations which provide from 0.1 to 1 g of the active ingredient per unit dose are preferred and are conventionally prepared as tablets, lozenges, capsules, powders, aqueous or oily suspensions, syrups, elixirs, and aqueous solutions. Preferred oral formulations are in the form of tablets, capsules, and may contain conventional excipients such as binding agents, (e.g., syrup, acacia, gelatin, sorbitol, tragacanth, or polyvinylpyrrolidone), fillers (e.g. lactose, sugar, maize-starch, calcium phosphate, sorbitol or glycine), lubricants (e.g. magnesium stearate, talc, polyethylene glycol or silica), disintegrants (e.g. starch) and wetting agents (e.g. sodium lauryl sulfate). Solutions or suspensions of a formula I compound with conventional pharmaceutical vehicles are employed for parenteral compositions such as an aqueous solution for intravenous injection or an oily suspension for intramuscular injection. Such compositions having the desired clarity, stability and adaptability for parenteral use are obtained by dissolving from about 0.1% to 10% by weight of the active compound in water or a vehicle consisting of a polyhydric aliphatic alcohol such as glycerine, propylene glycol, and the polyethylene glycols or mixtures thereof. The polyethylene glycols consist of a mixture of non-volatile, usually liquid, polyethylene glycols which are soluble in both water and organic liquids and which have molecular weights from about 200 to 1500.

## Claims

**1.** A compound of formula I or a pharmaceutically acceptable salt thereof

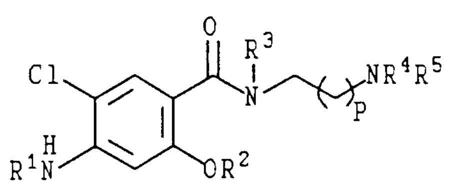

I

wherein

| | |
|---|---|
| p | is 1 to 3; |
| $R^1$ | is hydrogen or an acyl group $R^6CO$-, in which $R^6$ is $C_{1-6}$ alkyl, $C_{3-7}$ cycloalkyl, $C_{2-6}$ alkenyl, aryl or radical of the formula |

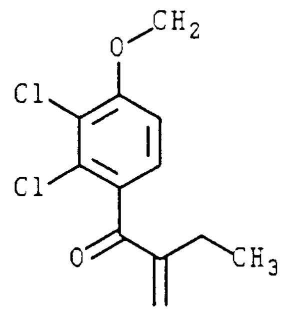 ;

$R^3$ is hydrogen or W, where W is a radical of the formula

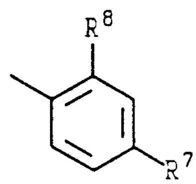

in which $R^7$ and $R^8$ each are independently $CF_3$, H or $-NO_2$, provided at least either $R^7$ or $R^8$ is $-NO_2$;

$R^2$ is W or a radical of the formula

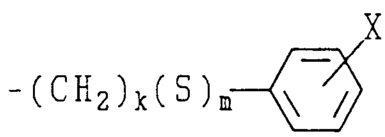

in which X is hydrogen or a halogen, k is 2 or 3, and m is 0 or 1; and

$R^4$ and $R^5$ each are independently $C_{1-6}$ alkyl.

2. A compound of formula I as claimed in Claim 1 in which p is 1, $R^2$ and $R^3$ are simultaneously W, or $R^3$ is hydrogen and $R^2$ is a radical of the formula

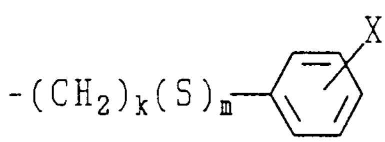

$R^1$ is hydrogen or an acyl group $R^6CO$- selected from

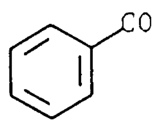 , 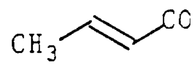 , $CH_3CO$ ,

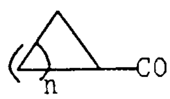 and 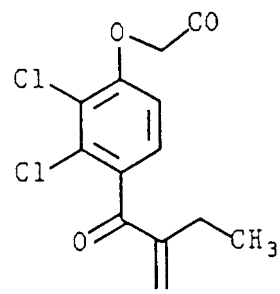

in which n is 1 to 4, X is chloro, and k and m are as defined in Claim 1.

3. The compound as claimed in Claim 2 which is 4-amino-5-chloro-N-[2-(diethylamino)ethyl]-N-[2-nitro-4-(trifluorormethyl)phenyl]-2-[(2-nitro-4-(trifluoromethyl)phenoxy]benzamide.

4. The compound as claimed in Claim 2 which is 4-amino-5-chloro-N-[2-(diethylamino)ethyl]-2-[3-(phenyl)-propoxy]benzamide.

5. The compound as claimed in Claim 2 which is 2-acetamido-5-chloro-N-[2-(diethylamino)ethyl]-2-[2-(phenylthio)ethoxy]benzamide.

6. The compound as claimed in Claim 2 which is 5-chloro-4-crotonylamino-N-[2-(diethylamino)ethyl]-2-[2-(phenylthio)ethoxy]benzamide.

7. A pharmaceutical formulation which comprises as an active ingredient a compound of formula I, as claimed in any one of Claims 1 to 6, associated with one or more pharmaceutically acceptable carriers, excipients or diluents therefor.

8. A process for preparing the pharmaceutical formulation of claim 7 which comprises incorporating at least one compound of any one of claims 1 to 6 into one or more pharmaceutically acceptable carriers, excipients or diluents.

9. The use of at least one compound of any one of claims 1 to 6 for preparing a pharmaceutical composition for reversing multidrug resistance of cancer cells to a cytotoxic drug.

10. A process for preparing the compounds of claims 1 to 6 which comprises
(a) reacting a compound of formula IV

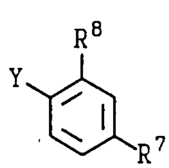

IV

in which Y is halogen, with a compound of formula III, XVIII or XIX,

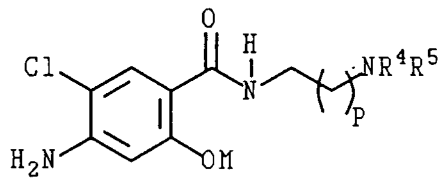

III

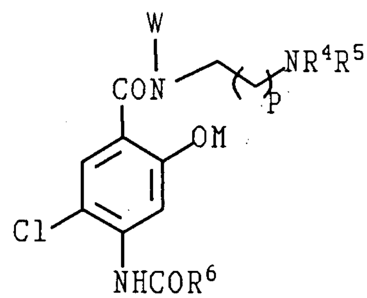

XVIII

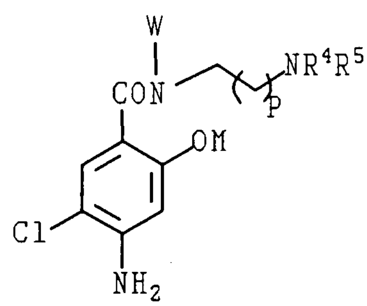

XIX

in which M is a cation, and isolating a compound of formula I; or

(b) reacting a compound of formula III, XVIII or XIX with a compound of the formula

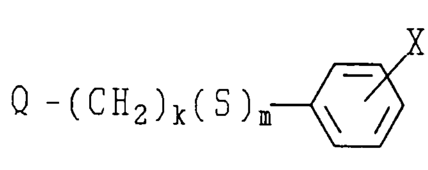

in which Q is a conventional leaving group; or

(c) reacting a compound of formula VI, XVI or XVII

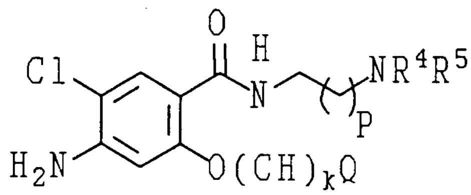

VI

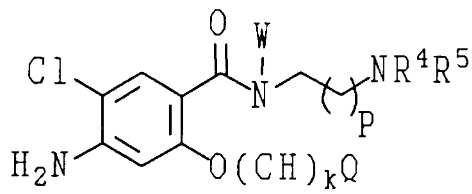

XVI

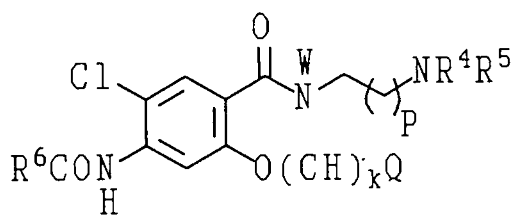

XVII

with a compound of the formula

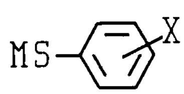

; or

(d) condensing a compound of formula XIV

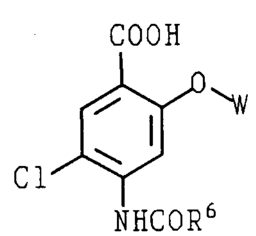

XIV

with $H_2NCH_2(CH_2)_pNR^4R^5$ or $HWNCH_2(CH_2)_pNR^4R^5$.